# FASCICULE DE BREVET EUROPEEN

(11) **EP 2 446 826 B1**
(45) Date de publication et mention de la délivrance du brevet: **08.07.2015**
(21) Numéro de dépôt: 11186204.1
(22) Date de dépôt: 21.10.2011
(51) Int. Cl.: A61B 8/12, A61B 5/00

(54) **Protection stérile à guides de lumière pour sonde médicale et procédé de réalisation associé**
Steriler Schutz mit Lichtführungen für eine medizinische Sonde, und zugehöriges Herstellungsverfahren
Sterile protection with light guides for a medical probe and related execution method

(30) Priorité: 27.10.2010 FR 1058834
(43) Date de publication de la demande: 02.05.2012
(73) Titulaire: Commissariat à l'Énergie Atomique et aux Énergies Alternatives, 75015 Paris (FR)
(72) Inventeur: Boutet, Jérôme, 38640 Claix (FR); Debourdeau, Mathieu, 38830 Saint Pierre D'Allevard (FR)
(74) Mandataire: Bréda, Jean-Marc

(56) Documents cités:
- US-A- 5 478 338
- US-A1- 2002 143 275
- JIANG Z ET AL: "Trans-rectal ultrasound-coupled near-infrared optical tomography of the prostate Part II: Experimental demonstration", 27 octobre 2008 (2008-10-27), OPTICS EXPRESS 20081027 OPTICAL SOCIETY OF AMERICA US, VOL. 16, NR. 22, PAGE(S) 17505 - 17520, XP002646498, * Section 2.1 Development of a TRUS-coupled NIR applicator for a trans-rectal optical tomography * * figure 1 *

## Description

Le domaine de l'invention est celui des sondes médicales dites multimodales utilisés pour certains types de diagnostic. Ces sondes combinent l'emploi d'une imagerie optique et ultrasonore.

Récemment, l'imagerie dite multi-modalité, combinant l'acquisition de différents types d'image, a connu un essor important dans le domaine du diagnostic médical. En effet, elle permet d'obtenir à la fois des informations morphologiques et fonctionnelles. Les premières sont obtenues par l'utilisation de sondes à rayons X ou à ultrasons, les secondes par l'emploi de la tomographie à émission de positron ou « PET » ou de l'imagerie à résonance magnétique ou « IRM » ou par l'utilisation de l'imagerie par fluorescence. En particulier, le couple des techniques ultrasons-optique semble particulièrement pertinent pour certaines applications comme la mammographie ou la détection d'anomalies de la prostate car ces techniques sont compatibles en termes de coût, de compacité et de profondeur de pénétration.

De nombreuses équipes de recherche développent actuellement des instruments basés sur ces deux modalités d'imagerie soit pour des applications de diagnostic, soit pour des applications de traitement par photothérapie.

A l'heure actuelle, le principal outil de diagnostic pour le cancer de la prostate consiste à réaliser plusieurs biopsies à l'aide en se guidant à l'aide d'une sonde ultrasonore endorectale. Pour l'examen, le praticien enfile une protection stérile autour de la sonde permettant de réduire le risque d'infections nosocomiales. Cette protection peut-être, selon les praticiens, un simple préservatif, ou bien un gant stérile dédié. En France, chaque centre hospitalier semble suivre sa propre procédure de stérilisation de ces sondes.

Cependant, durant le protocole de biopsie guidée par ultrasons, le risque de perforation de la protection stérile est de l'ordre de 9%. Il n'est pas négligeable et conduit à des risques d'infection. On se reportera à l'étude de J. Masood, S. Voulgaris, O. Awogu, C. Younis, A.J. Ball, et T.W. Carr, "Condom perforation during transrectal ultrasound guided (TRUS) prostate biopsies: a potential infection risk," International Urology and Nephrology, vol. 39, 2007, p. 1121―1124 pour toutes informations sur ce problème.

D'autre part, les gants stériles existants actuellement ne sont pas prévus pour être compatibles avec des mesures en fluorescence. En particulier, les problèmes suivants se posent:
- Les colorants présents dans le matériau des protections absorbent une partie de la lumière d'absorption et d'excitation ;
- Le matériau de la protection peut également induire un signal de fluorescence parasite dans les longueurs d'onde utilisées ;
- Il peut également, dans certaines conditions, se produire une détérioration de la protection sous l'effet de la lumière laser. On peut citer l'exemple des méthodes de photo-ablation qui requièrent une puissance laser importante pour découper les tissus.

Or, on assiste depuis quelques années à un durcissement des critères de stérilisation des outils utilisés dans le milieu médical, ce qui entraîne une tendance vers l'utilisation de gants stériles plus épais et donc absorbants plus la lumière.

L'intérêt de disposer d'une sonde permettant de réaliser une échographie et une imagerie de fluorescence est aujourd'hui admis, et certains se sont orientés sur des dispositifs dédiés, intégrant, dans une même sonde, les modalités optique et acoustique pour l'échographie. L'ajout de la modalité optique est susceptible de nuire à l'étanchéité de la partie ultrasonore de la sonde. En effet, la présence de fibres optiques affleurant la surface de la sonde peut engendrer des fuites dans l'étanchéité de l'ensemble du dispositif. Par ailleurs, inclure des fibres optiques dans une sonde ultrasonore représente un véritable défi technique car celles-ci ont déjà été optimisées sur le plan de l'encombrement. Une telle modification implique de revoir complètement le design des sondes actuelles et de changer le parc de sondes existantes, ce qui n'est pas souhaité par les praticiens.

Pour résoudre les problèmes posés à la fois par la bimodalité et par la stérilisation de la sonde, plusieurs solutions ont été proposées.

Une équipe américaine propose une sonde couplant optique et ultrasons pour le diagnostic du cancer de la prostate. Il s'agit de l'équipe de Z. Jiang, G. Holyoak, K. Bartels, J. Ritchey, G. Xu, C. Bunting, G. Slobodov, et D. Piao, "In vivo trans-rectal ultrasound-coupled optical tomography of a transmissible venereal tumor model in the canine pelvic canal," JOURNAL OF BIOMEDICAL OPTICS, vol. 14, Mai. 2009. Cependant, cette sonde ne résout pas le problème de la stérilisation après usage.

Le brevet U.S. Patent US5283722 de K.M. Peter et R.Trow, « Surgical-Type Glove and illuminator Assembly » propose un gant stérile destiné au chirurgien et équipé d'une source de lumière pour éclairer le champ de travail chirurgical.

Une autre équipe propose une sonde optique fibrée dont une partie est jetable, cette sonde est destinée à mesurer certaines caractéristiques du sang comme le pH au travers d'un cathéter. On se reporter à la publication de W.W. Miller, M. Yafuso, C.F. Yan, H.K. Hui et S. Arick intitulée « Performance of an in-vivo continuous blood-gas monitor with disposable probe » Clinical Chemistry, Vol. 33, 1987, p. 1538.

Le brevet US 4 870 952 de M. Martinez intitulé « Fiber Optic Illuminator for Use in Surgery » décrit une sonde optique partiellement jetable pour des applications chirurgicales.

On note également qu'une équipe des Pays-Bas propose une seringue à biopsie jetable pour l'examen de la prostate. On se reportera à la publication de C.C. Schulman intitulée « Transrectal prostatic biopsy » International Urology and Nephrology, Vol. 2, 1970, p. 157-161 sur ce sujet.

Le document US2002/143275 divulgue une protection stérile souple pour sonde médicale de type transrectale (voir paragraphes [0010] et [0038]), ladite protection comprenant un capuchon fin en latex de forme adaptée pour protéger totalement la sonde médicale en utilisation (voir paragraphe [0056] et fig. 5A).

Le document US5478338 divulgue la combinaison des ultrasons et l'imagerie optique pour le diagnostique du cancer de prostate est connue dans l'état de la technique (voir figure 2 dans D2).

Aucune de ces sondes ne répond complètement aux problèmes posés. L'objet de l'invention est de proposer un dispositif simple qui réponde parfaitement au double souci de la bimodalité et de la stérilisation. Le dispositif proposé est une protection aussi appelée « gant » ou « chaussette » stérile jetable ou stérilisable équipée de fibres optiques. Cette protection est enfilable sur une sonde ultrasonore. Elle assure donc à la fois la bimodalité fonctionnelle et la stérilisation de la sonde, ce qui est le but recherché.

Plus précisément, l'invention a pour premier objet une protection stérile pour sonde médicale, ladite protection comprenant un capuchon fin en latex de forme adaptée pour protéger totalement la sonde médicale en utilisation, caractérisé en ce que, dans ledit capuchon est inclus au moins une fibre optique, ladite fibre optique comprenant un moyen de couplage optique à l'extrémité libre du capuchon et agencée de façon à amener ou à recueillir de la lumière dans la zone de mesure de la sonde médicale.

Avantageusement, les fibres optiques sont des fibres en plastique, le latex est dépourvu de chromophores et l'épaisseur de latex du capuchon est comprise entre 50 microns et 150 microns.

L'invention a pour second objet un banc de diagnostic comprenant une sonde médicale de type transrectale protégée par une protection stérile telle que définie ci-dessus, ledit banc comprenant au moins :
une source optique agencée de façon à adresser séquentiellement un premier faisceau de fibres optiques, ledit premier faisceau étant couplé au moyen d'une couronne de connexion optique équipée de férules à un premier ensemble de fibres optiques incluses dans la protection ;
des capteurs optiques reliés à un second faisceau de fibres optiques, ledit second faisceau étant couplé au moyen de ladite couronne de connexion optique à un second ensemble de fibres optiques incluses dans la protection.

Avantageusement, la sonde médicale est une sonde endorectale ou une sonde d'échographie vaginale.

Enfin l'invention a pour troisième objet le procédé de réalisation de ladite protection stérile, le procédé comporte alors les étapes suivantes :
- 1 ^{ère} étape : Positionner des fibres optiques autour d'un moule ayant la même forme que la sonde ultrasonore et maintenir celles-ci en place à l'aide d'un ou de plusieurs anneaux souples ;
- 2^{ème} étape : Immerger l'ensemble moule-fibres-anneaux dans un bain de latex ;
- 3^{ème} étape : Attendre qu'un film en latex se dépose sur le moule sous forme gélifiée. Retirer l'ensemble moule-fibres-anneaux du bain de latex. Laisser sécher le revêtement en latex ;
- 4^{ème} étape : Effectuer si nécessaire un ou plusieurs trempages supplémentaires afin d'augmenter l'épaisseur du revêtement de latex ;
- 5^{ème} étape : Une fois l'épaisseur de latex souhaitée atteinte, enrouler l'extrémité ouverte du film en latex venant d'être moulé pour former une ceinture d'épaisseur plus importante.

L'invention sera mieux comprise et d'autres avantages apparaîtront à la lecture de la description qui va suivre donnée à titre non limitatif et grâce aux figures annexées parmi lesquelles :
Les figures 1 et 2 représentent les niveaux de fluorescence parasites en fonction du temps générés par les chromophores présents dans une protection stérile du commerce et l'efficacité de transmission d'un signal utile provenant d'une inclusion fluorescente au travers d'une protection stérile ;
La figure 3 représente en vue de face et en vue de coupe une protection stérile selon l'invention ;
La figure 4 représente un banc d'analyse ou de diagnostic comprenant une sonde médicale de type transrectale protégée par une protection stérile selon l'invention.

La protection stérile selon l'invention permet d'acheminer la lumière d'une source vers l'organe étudié, en utilisant une fibre optique, dite fibre optique d'excitation, et de ramener la lumière de fluorescence ou de diffusion détectée vers au moins un détecteur, en utilisant une fibre optique dite fibre de fluorescence ou fibre de réception. Il s'agit d'une protection réalisée en un matériau souple et élastique, permettant de se conformer à la forme de la sonde endorectale utilisée. Ainsi, on peut coupler aisément la protection sur une sonde endorectale acoustique existante.

Les niveaux d'absorption et de signal parasite générés par deux modèles de protections stériles en latex du commerce utilisés pour protéger les sondes transectales ont été mesurés en suivant le protocole expérimental décrit par J. Boutet, L. Herve, M. Debourdeau, L. Guyon, P. Peltie, J.M. Dinten, L. Saroul, F. Duboeuf, et D. Vray, "Bimodal ultrasound and fluorescence approach for prostate cancer diagnosis," Journal of Biomedical Optics, vol. 14, 2009, p. 064001, ce protocole étant décrit dans le paragraphe 2. On ajoute à ce protocole la présence d'une protection stérile représentative de ce que l'on peut trouver dans le commerce.

Le matériau comprenant l'enveloppe de la protection doit être fin et souple. Il ne doit pas comporter une très faible concentration en chromophores ou en fluorophores, afin de ne pas affecter ses propriétés de transmission optique. Le matériau préféré est le Latex, qui satisfait à ces conditions, encore faut-il déterminer une épaisseur optimale : suffisante pour assurer une bonne résistance au déchirement, tout en étant suffisamment faible pour garantir une bonne transmission du signal optique, et un faible niveau d'émission de signal de fluorescence parasite. Par signal de fluorescence parasite, on entend un signal optique produit par le matériau en réponse à une excitation lumineuse.

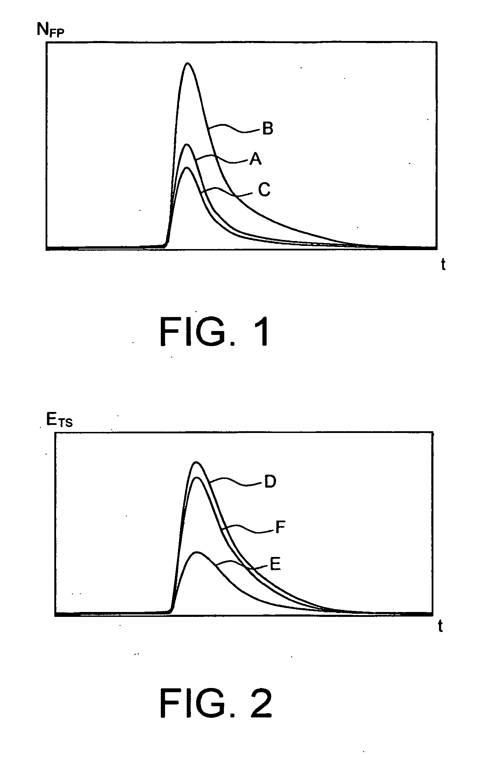

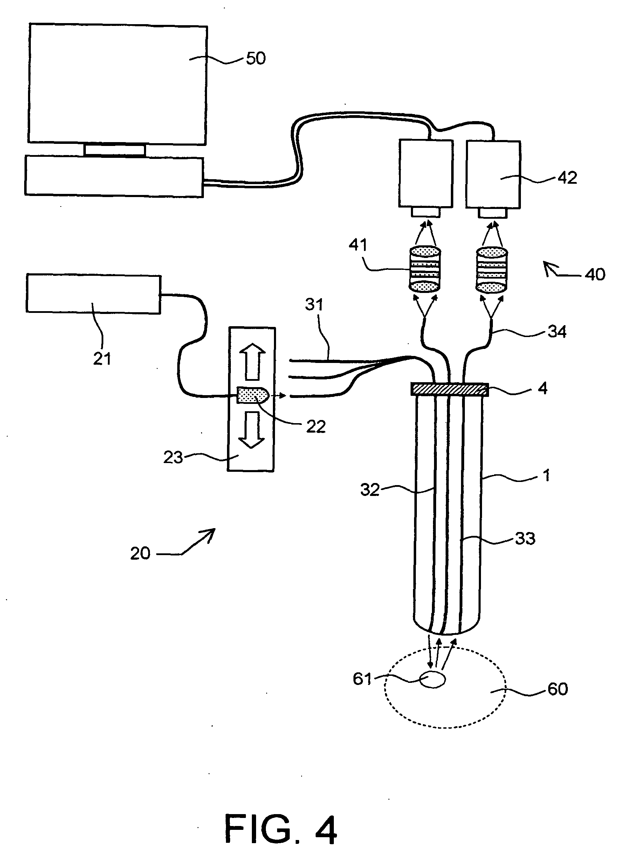

Le premier paramètre illustré en figure 1 représente les niveaux de fluorescence parasites N_{FP} en fonction du temps t générés par les chromophores présents dans une protection stérile du commerce en réponse à une impulsion lumineuse brève, c'est-à-dire dont la durée est généralement comprise entre 100 fs et 10 ps. La courbe A représente la fluorescence parasite générée par une protection fine de 100 µm d'épaisseur, la courbe B représente la fluorescence parasite générée par une protection épaisse de 500 µm d'épaisseur. Enfin, la courbe C représente le signal de fluorescence parasite provenant de l'environnement dans lequel est plongé la sonde et obtenus avec la sonde nue sans protection. On constate que la présence d'une protection stérile épaisse, c'est-à-dire de l'ordre de 500 µm augmente sensiblement les niveaux de fluorescence parasite alors que la présence d'une protection fine, c'est-à-dire de l'ordre de 100 µm, ne change pas sensiblement ces niveaux. Lors de ces essais, la sonde n'est pas mise à proximité d'un fluorophore, afin d'être certain que le signal mesuré est bien un signal d'autofluorescence généré par le matériau, en l'occurrence le Latex. Le dispositif expérimental mis en oeuvre lors de ces essais est similaire à celui présenté sur la figure 4, exception faite de l'inclusion fluorescente 61 et du fantôme de tissu 60.

Le second paramètre illustré en figure 2 représente l'intensité E_{Ts} d'un signal utile provenant d'une inclusion fluorescente, représentant une tumeur marquée par un fluorophore, ce signal traversant une protection stérile, en réponse à une impulsion lumineuse brève, de même que lors de l'exemple précédent. La courbe D représente l'intensité du signal de fluorescence, en fonction du temps, à travers une protection fine de 100 µm d'épaisseur, la courbe E représente l'intensité de fluorescence, en fonction du temps, à travers une protection épaisse de 500 µm d'épaisseur. Enfin, la courbe F représente l'intensité du signal de fluorescence de l'inclusion fluorescente, en fonction du temps, sans protection. On constate, que la présence d'une protection stérile épaisse, c'est-à-dire de l'ordre de 500 µm, diminue sensiblement l'intensité du signal de fluorescence alors que la présence d'une protection fine ne la modifie quasiment pas, cela en comparaison avec l'intensité du signal de fluorescence sans la protection. Le dispositif expérimental mis en oeuvre lors de ces essais est similaire à celui présenté sur la figure 4, en utilisant une inclusion fluorescente 61 à proximité de la sonde.

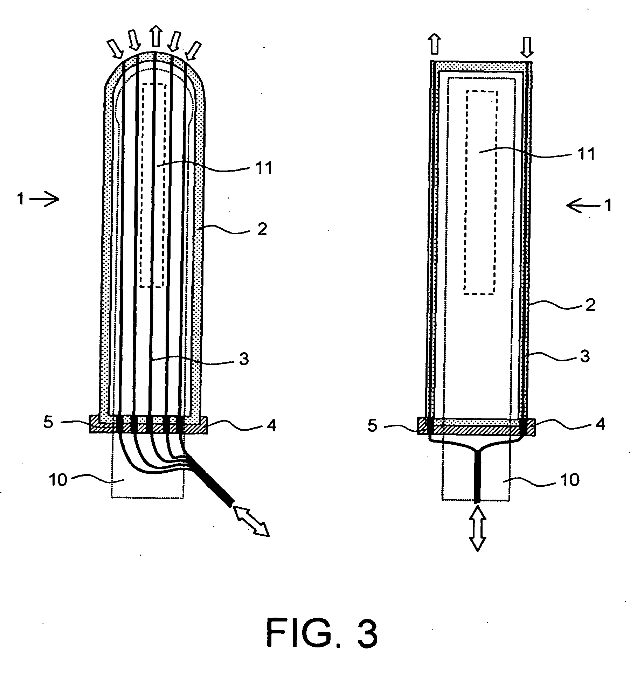

La figure 3 représente en vue de face et en vue de coupe un exemple de protection stérile 1 selon l'invention. Pour des raisons de clarté des dessins, les dimensions et les épaisseurs indiquées ne sont pas représentatives des dimensions réelles de la protection. Sur ces vues, est également représentée en traits pointillés la sonde ultrasonore 10 protégée ainsi que son transducteur ultrasonore 11.

La protection comprend un capuchon en latex 2 de forme oblongue et des fibres optiques 3 incluses dans le film de latex qui assure l'émission de la lumière venant d'une source extérieure non représentée sur la figure 3 et la réception de la lumière émise par le tissu biologique, en réponse à la lumière d'excitation. Le parcours de la lumière dans la protection est représenté par des flèches épaisses blanches sur la figure 3. Préférentiellement, les fibres d'excitation et de réception sont distinctes. On peut ainsi optimiser de façon indépendante les voies d'excitation et de réception. Les fibres sont raccordées à une couronne de connexion optique 4 équipée de férules 5. Cette couronne est indépendante de la protection 1 et peut s'en détacher.

Les dimensions typiques de la protection stérile selon l'invention sont un diamètre d'environ 2 centimètres et une longueur d'environ 15 centimètres.

Il est préférable que le film de latex ait une épaisseur fine de l'ordre de 100 microns afin de diminuer les niveaux de fluorescence parasites et d'augmenter l'efficacité de transmission du signal utile comme il a été décrit plus haut, en relation avec les figures 1 et 2. Un bon compromis est que l'épaisseur du film soit comprise entre 50 et 150 microns, ce qui permet également une bonne résistance à la perforation. Il est préférable d'éviter d'utiliser des matériaux tels que le nitrile ou le polyuréthane en raison de leur teneur élevée en chromophores susceptibles de perturber la mesure finale. Le Latex est du Latex naturel ou synthétique, le latex naturel présentant moins de risque de déchirure.

La longueur des fibres optiques dépend bien entendu de la taille de la sonde à protéger. Classiquement, la longueur des fibres optiques est de l'ordre de 20 centimètres.

Les fibres d'émission ou d'excitation peuvent être de type gaine en polyimide avec un coeur en silice. A titre d'exemple, les différents paramètres géométriques peuvent être :
Diamètre du coeur : 62.5 microns
Diamètre de la gaine : 155 microns
Ouverture numérique : 0.27
Rayon de courbure minimum : 17 millimètres

Les fibres de réception peuvent être de type plastique. Ces fibres présentent de nombreux avantages. Elles sont souples et ont une ouverture numérique importante, de l'ordre de 0.5 permettant de collecter un flux lumineux important. A titre d'exemple, les différents paramètres géométriques peuvent être :
Diamètre du coeur : 1 millimètre
Ouverture numérique : 0.46
Rayon de courbure minimum : 10 millimètres

Le nombre de fibres d'excitation et de réception dépend de l'application envisagée. Plus le nombre de fibres de détection est élevée, meilleure est la résolution. En pratique, le nombre de fibres est limitée à quelques unités, et varie généralement entre 2 et 20.

A titre d'exemple, le procédé de réalisation de la protection selon l'invention comporte les étapes suivantes :
- 1 ^{ère} étape : Positionner des fibres optiques autour d'un moule ayant la même forme que la sonde ultrasonore d'utilisation et maintenir celles-ci en place à l'aide d'un ou de plusieurs anneaux souples ;
- 2^{ème} étape : Immerger l'ensemble moule-fibres-anneaux dans un bain de latex ;
- 3^{ème} étape : Attendre qu'un film en latex se dépose sur le moule sous forme gélifiée. Retirer l'ensemble moule-fibres-anneaux du bain de latex. Laisser sécher le revêtement en latex ;
- 4^{ème} étape : Effectuer si nécessaire un ou plusieurs trempages supplémentaires afin d'augmenter l'épaisseur du revêtement de latex ;
- 5^{ème} étape : Une fois l'épaisseur de latex souhaitée atteinte, enrouler l'extrémité ouverte du film en latex venant d'être moulé pour former une ceinture d'épaisseur plus importante.

Le mode d'emploi de la protection selon l'invention est très simple. Il comporte les étapes suivantes :
- 1 ^{ère} étape : Glisser la couronne optique autour de la sonde ultrasonore transrectale :
- 2^{ème} étape : Sortir une protection stérile de son conditionnement stérile ;
- 3^{ème} étape : Glisser la protection sur la sonde ultrasonore transrectale ;
- 4^{ème} étape : Connecter les fibres de la protection sur celles de la couronne. Il est préférable que celle-ci comporte un détrompeur mécanique ou des repères visuels ;
- 5^{ème} étape : réaliser l'examen du patient en bi-modalité optique et ultrasonore ;
- 6^{ème} étape : Après l'examen, débrancher les fibres de la protection de la couronne optique ;
- 7^{ème} étape : Retirer la protection et l'éliminer ;
- 8^{ème} étape : Retirer la couronne optique de la sonde ultrasonore.

Dans une variante, la protection peut être réutilisable. Elle est alors nécessairement constituée d'éléments résistants à un passage en autoclave pour stérilisation.

A titre d'exemple, la figure 4 représente un banc d'analyse ou de diagnostic comprenant une sonde médicale protégée par une protection stérile selon l'invention. A titre d'exemple, la sonde peut être une sonde endorectale ou une sonde d'échographie vaginale. On peut, bien entendu, l'utiliser pour l'analyse d'autres cavités naturelles.

Seule la partie optique 20 de ce banc est représentée sur la figure 4. Elle comprend :
- une source optique 21, continue ou impulsionnelle, agencée de façon à adresser séquentiellement un premier faisceau de fibres optiques 31 appartenant à la protection stérile 1, ledit premier faisceau 31 étant couplé au moyen de la couronne de connexion optique 4 équipée de férules à un premier ensemble de fibres optiques d'émission 32 incluses dans la protection 1. Dans le cas de la figure 4, le balayage séquentiel est obtenu au moyen d'un dispositif optique de couplage 22 monté sur des moyens de translation 23. D'autres dispositions optiques sont possibles. Dans le dispositif expérimental mis en oeuvre par les inventeurs, la source de lumière 21 est un Laser pulsé Titane-Saphir, produisant des impulsions de 50 femtosecondes à 770 nm à une fréquence de 80 MHz. La puissance moyenne est de 10 mW. La lumière émise est filtrée par un filtre passe-bande centré sur la longueur d'onde de 770 nm. Le dispositif optique de couplage 22 est une lentille de grandissement 10x montée sur une platine de déplacement 23.
- des capteurs optiques 40 reliés à un second faisceau de fibres optiques 34, ledit second faisceau étant couplé au moyen de la couronne de connexion optique 4 à un second ensemble de fibres optiques de réception 33 incluses dans la protection 1. Généralement, les capteurs 40 comprennent un ensemble optique 41 éventuellement muni de filtres optiques et un boîtier photosensible 42. L'ensemble optique 41 peut par exemple comporter une lentille de grandissement 6.3x ainsi que des filtres optiques, permettant notamment de s'affranchir des longueurs d'ondes de la lumière d'excitation. Le boîtier photosensible 42 peut être une caméra intensifiée rapide. L'ensemble des informations issues des différents capteurs optiques est traité et affiché par des moyens informatiques 50 dédiés.

La lumière issue des fibres est émise vers des tissus biologiques inspectés ou des fantômes simulant ces tissus 60, ces tissus émettant en réponse une lumière dite rediffusée, dont l'analyse permet d'obtenir des informations sur la présence de lésions ou de tumeurs 61. Lors des essais expérimentaux réalisés sur un fantôme, la tumeur est simulée par une inclusion fluorescente que l'on dispose dans le fantôme. Le lumière rediffusée est par exemple une lumière de fluorescence provenant de marqueurs fluorescents préalablement introduits.

La particularité de cette protection est d'être constituée exclusivement d'éléments à bas coûts permettant de ne l'utiliser qu'une seule fois, la protection n'étant plus qu'un simple consommable jetable.

Les avantages d'une protection selon l'invention sont :
- Obtention d'une hygiène optimale ;
- Suppression de l'emploi d'une procédure complexe de désinfection chimique ;
- Compatibilité avec le parc de sondes ultrasonores existantes. Les praticiens n'ont pas besoin de racheter une sonde bi-modalité spécifique ;
- Optimisation de la reproductibilité des mesures optiques. Au début de l'examen, l'interface des surfaces optiques est parfaitement propre ;
- Meilleur couplage optique puisque les fibres sont directement en contact avec les tissus humains, le film de latex de la protection ne perturbe plus la mesure.

## Revendications

1. Protection stérile souple (1) pour sonde médicale de type transrectale, ladite protection comprenant un capuchon (2) fin en latex de forme adaptée pour protéger totalement la sonde médicale en utilisation, **caractérisé en ce que**, dans ladite enveloppe est incluse au moins une fibre optique (3, 32, 33), ladite fibre optique comprenant un moyen de couplage optique (4, 5) à l'extrémité libre du capuchon et étant agencée pour amener ou pour recueillir de la lumière dans la zone de mesure de la sonde médicale.

2. Protection stérile pour sonde médicale selon la revendication 1, **caractérisé en ce que** les fibres optiques sont des fibres en plastique.

3. Protection stérile pour sonde médicale selon la revendication 1, **caractérisé en ce que** le moyen de couplage optique est apte à connecter optiquement ladite fibre à une source de lumière ou à un détecteur de lumière.

4. Protection stérile pour sonde médicale selon la revendication 1, **caractérisé en ce que** l'épaisseur de latex du capuchon est comprise entre 50 microns et 150 microns.

5. Banc d'analyse ou de diagnostic comprenant une sonde médicale de type transrectale protégée par une protection stérile (1) selon l'une des revendications précédentes, **caractérisé en ce que** ledit banc comprend au moins :
une source optique (21, 22, 23) agencée de façon à adresser séquentiellement un premier faisceau (31) de fibres optiques, ledit premier faisceau étant couplé au moyen d'une couronne (4) de connexion optique équipée de férules à un premier ensemble de fibres optiques (32) incluses dans la protection ;
des capteurs optiques (40, 41, 42) reliés à un second faisceau (34) de fibres optiques, ledit second faisceau étant couplé au moyen de ladite couronne de connexion optique à un second ensemble (33) de fibres optiques incluses dans la protection.

6. Banc d'analyse ou de diagnostic selon la revendication 5, **caractérisé en ce que** la sonde médicale est une sonde endorectale ou une sonde d'échographie vaginale.

7. Procédé de réalisation d'une protection stérile selon les revendications 1 à 4, **caractérisé en ce que** le procédé comporte les étapes suivantes :
- 1^{ère} étape : Positionner des fibres optiques autour d'un moule ayant la même forme que la sonde ultrasonore et maintenir celles-ci en place à l'aide d'un ou de plusieurs anneaux souples ;
- 2^{ème} étape : Immerger l'ensemble moule-fibres-anneaux dans un bain de latex ;
- 3^{ème} étape : Attendre qu'un film en latex se dépose sur le moule sous forme gélifiée. Retirer l'ensemble moule-fibres-anneaux du bain de latex. Laisser sécher le revêtement en latex ;
- 4^{ème} étape : Effectuer si nécessaire un ou plusieurs trempages supplémentaires afin d'augmenter l'épaisseur du revêtement de latex ;
- 5^{ème} étape : Une fois l'épaisseur de latex souhaitée atteinte, enrouler l'extrémité ouverte du film en latex venant d'être moulé pour former une ceinture d'épaisseur plus importante.

## Patentansprüche

1. Steriler flexibler Schutz (1) für eine medizinische Sonde des Transrektaltyps, wobei der Schutz eine Endkappe (2) aus Latex in einer Form umfasst, die zum totalen Schützen der medizinischen Sonde beim Gebrauch gestaltet ist, **dadurch gekennzeichnet, dass** die Hülle wenigstens eine Lichtleitfaser (3, 32, 33) umschließt, wobei die Lichtleitfaser ein Mittel (4, 5) zum optischen Koppeln mit dem freien Ende der Kappe hat und zum Bringen oder Sammeln von Licht in der Messzone der medizinischen Sonde ausgelegt ist.

2. Steriler Schutz für eine medizinische Sonde nach Anspruch 1, **dadurch gekennzeichnet, dass** die Lichtleitfasern Kunststofffasern sind.

3. Steriler Schutz für eine medizinische Sonde nach Anspruch 1, **dadurch gekennzeichnet, dass** das Mittel zum optischen Koppeln die Faser optisch mit einer Lichtquelle oder einem Lichtdetektor verbinden kann.

4. Steriler Schutz für eine medizinische Sonde nach Anspruch 1, **dadurch gekennzeichnet, dass** die Latexdicke der Kappe zwischen 50 Mikron und 150 Mikron liegt.

5. Analyse- oder Diagnostikanlage, die eine medizinische Sonde des Transrektaltyps umfasst, geschützt durch einen sterilen Schutz (1) nach einem der vorherigen Ansprüche, **dadurch gekennzeichnet, dass** die Anlage wenigstens Folgendes umfasst:
eine optische Quelle (21, 22, 23) zum sequentiellen Adressieren eines ersten Strahls (31) von optischen Fasern, wobei der erste Strahl mittels eines mit Muffen ausgestatteten optischen Verbindungskranzes (4) mit einem ersten Satz von in dem Schutz eingeschlossenen Lichtleitfasern (32) gekoppelt ist;
optische Sensoren (40, 41, 42), die mit einem zweiten Strahl (34) von optischen Fasern verbunden sind, wobei der zweite Strahl mittels des optischen Verbindungskranzes mit einem zweiten Satz (33) von in dem Schutz eingeschlossenen Lichtleitfasern gekoppelt ist.

6. Analyse- und Diagnostikanlage nach Anspruch 5, **dadurch gekennzeichnet, dass** die medizinische Sonde eine Endorektalsonde oder eine Vaginalechographie-Sonde ist.

7. Verfahren zur Herstellung eines sterilen Schutzes nach den Ansprüchen 1 bis 4, **dadurch gekennzeichnet, dass** das Verfahren die folgenden Schritte beinhaltet:
- 1. Schritt: Positionieren der optischen Fasern um eine Form mit derselben Gestalt wie die Ultraschallsonde, und Festhalten derselben mit Hilfe von einem oder mehreren flexiblen Ringen;
- 2. Schritt: Eintauchen der Form-Faser-Ring-Baugruppe in ein Latexbad;
- 3. Schritt: Warten, bis sich ein Latexfilm in einer gelierten Form auf die Form abgesetzt hat; Herausnehmen der Form-Faser-Ring-Baugruppe aus dem Latexbad; Trocknenlassen des Latexüberzugs;
- 4. Schritt: bei Bedarf Durchführen von einem oder mehreren ergänzenden Eintauchvorgängen, um die Dicke des Latexüberzugs zu erhöhen;
- 5. Schritt: nach dem Erreichen der gewünschten Latexdicke Aufrollen des offenen Endes des soeben geformten Latexfilms, um einen Gürtel mit einer größeren Dicke zu bilden.

## Claims

1. Flexible sterile protection (1) for a medical probe of the transrectal type, the protection comprising a fine latex cap (2) which has a shape capable of completely protecting the medical probe during use, **characterised in that** at least one optical fibre (3, 32, 33) is included in the cover, the optical fibre comprising an optical coupling means (4, 5) at the free end of the cap and being arranged so as to bring or acquire light in the measuring zone of the medical probe.

2. Sterile protection for a medical probe according to claim 1, **characterised in that** the optical fibres are plastics material fibres.

3. Sterile protection for a medical probe according to claim 1, **characterised in that** the optical coupling means is capable of optically connecting the fibre to a source of light or a light detector.

4. Sterile protection for a medical probe according to claim 1, **characterised in that** the thickness of the latex of the cap is between 50 micrometres and 150 micrometres.

5. Diagnostic or analysis bench comprising a medical probe of the transrectal type which is protected by a sterile protection (1) according to any one of the preceding claims, **characterised in that** the bench comprises at least:
one optical source (21, 22, 23) which is arranged so as to sequentially address a first bundle (31) of optical fibres, said first bundle being coupled by means of an optical connection ring (4) which is provided with ferrules to a first assembly of optical fibres (32) included in the protection;
optical sensors (40, 41, 42) which are connected to a second bundle (34) of optical fibres, the second bundle being coupled by means of the optical connection ring to a second assembly (33) of optical fibres included in the protection.

6. Diagnostic or analysis bench according to claim 5, **characterised in that** the medical probe is an endorectal probe or a vaginal echography probe.

7. Method for producing a sterile protection according to claims 1 to 4, **characterised in that** the method comprises the following steps:
- 1 st step: positioning optical fibres around a mould which has the same shape as the ultrasound probe and holding them in place using one or more flexible rings;
- 2nd step: immersing the mould/fibre/rings assembly in a latex bath;
- 3rd step: waiting until a latex film is deposited on the mould in gel form. Withdrawing the mould/fibre/rings assembly from the latex bath. Allowing the latex coating to dry;
- 4th step: where applicable, carrying out one or more additional dipping operations in order to increase the thickness of the latex coating;
- 5th step: once the desired latex thickness has been reached, wrapping the open end of the film of latex which has been moulded in order to form a thicker belt.
